(19) Europäisches Patentamt
European Patent Office
Office européen des brevets

(11) Veröffentlichungsnummer: **0 657 433 A1**

# (12) EUROPÄISCHE PATENTANMELDUNG

(21) Anmeldenummer: **94118678.5**

(22) Anmeldetag: **28.11.94**

(51) Int. Cl.[6]: **C07D 211/90**, C07D 401/12, C07D 405/12, A61K 31/44

(30) Priorität: **10.12.93 DE 4342195**

(43) Veröffentlichungstag der Anmeldung: **14.06.95 Patentblatt 95/24**

(84) Benannte Vertragsstaaten: **AT BE CH DE DK ES FR GB GR IE IT LI LU MC NL PT SE**

(71) Anmelder: **BAYER AG**
**D-51368 Leverkusen (DE)**

(72) Erfinder: **Junge, Bodo, Dr.**
**Spieckern 23**
**D-42399 Wuppertal (DE)**
Erfinder: **Meier, Heinrich, Dr.**
**The Entente 616,**
**5-15 Koyo-Cho,**
**Higashi-Nada-Ku**
**Kobe 658 (JP)**
Erfinder: **Hartwig, Wolfgang, Dr.**
**62 Rocky Rapids Road**
**Stamford,**
**Connecticut 06903 (US)**
Erfinder: **Schohe-Loop, Rudolf, Dr.**
**Arndtstrasse 10a**
**D-42237 Wuppertal (DE)**
Erfinder: **Ghao, Zhan, Dr.**
**2-16 Guang Ming Apartment**
**Beijing 100016 (CN)**
Erfinder: **Schmidt, Bernard, Dr.**
**In der Fuhr 8**
**D-51789 Lindlar (DE)**
Erfinder: **van der Staay, Franz-Josef, Dr.**
**Matthias-Claudius-Weg 15A**
**D-53797 Lohmar (DE)**
Erfinder: **de Jonge, Maarten, Dr.**
**Am Kreuzberg 9, D**
**D-51491 Overath (DE)**
Erfinder: **Schuurman, Teunis, Dr.**
**Rohrbergstrasse 20**
**D-53797 Lohmar (DE)**

(54) **Dihydropyridine mit substituierten Estern mit cerebraler Wirkung.**

(57) Neue Dihydropyridine mit substituierten Estern werden hergestellt indem man entsprechende Carbonsäuren gegebenenfalls über ein aktiviertes Derivat mit entsprechenden Alkoholen verestert oder den Substituenten in die Estergruppe einführt. Die Dihydropyridine können als Wirkstoffe in Arzneimitteln eingesetzt werden, insbesondere in cerebral wirksamen Mitteln.

EP 0 657 433 A1

Die Erfindung betrifft neue Dihydropyridine mit substituierten Estern, Verfahren zu ihrer Herstellung und ihre Verwendung in Arzneimitteln, insbesondere als cerebral wirksame Mittel.

Es ist bekannt, daß Nimodipin auch eine cerebrale Wirksamkeit besitzt [vgl. DOS 2 815 578]. In DOS 2 117 572 und DOS 1 963 188 werden bereits Dihydropyridine mit substituierten Estern durch allgemeine Substituentendefinitionen als Coronar-und Blutdruckmittel umfaßt. Die ausgewählten erfindungsgemäßen Verbindungen zeichnen sich durch ihre unerwartet vorteilhafte cerebrale Wirkung aus.

Die vorliegende Erfindung betrifft neue Dihydropyridine mit heterocyclisch substituierten Estern der allgemeinen Formel (I)

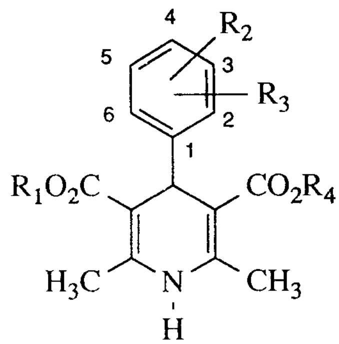

(I)

in welcher,

$R^1$ für geradkettiges oder verzweigtes Alkyl mit bis zu 6 Kohlenstoffatomen steht, das gegebenenfalls durch Cycloalkyl mit 3 bis 6 Kohlenstoffatomen substituiert ist, oder für Cycloalkyl mit 3 bis 6 Kohlenstoffatomen steht,

$R^2$ und $R^3$ gleich oder verschieden sind und für Halogen, Cyano oder Trifluormethyl stehen,

oder

$R^2$ oder $R^3$ für Wasserstoff steht,

und

$R^4$ für geradkettiges oder verzweigtes Alkyl mit bis zu 6 Kohlenstoffatomen steht, das durch geradkettiges oder verzweigtes Acyl oder Alkoxycarbonyl mit jeweils bis zu 4 Kohlenstoffatomen oder durch einen Rest der Formel

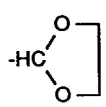
,
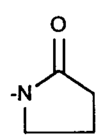
,
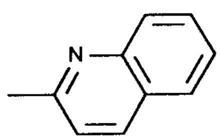
,
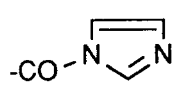
,

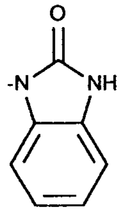
,
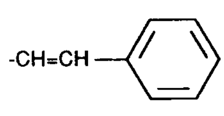
,
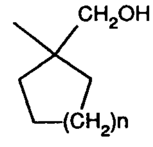

oder $-CO-NH-R^5$
substituiert ist,
worin

n eine Zahl 1, 2 oder 3 bedeutet
und

$R^5$ Cycloalkyl mit 3 bis 7 Kohlenstoffatomen bedeutet, oder geradkettiges oder verzweigtes Alkyl mit bis zu 6 Kohlenstoffatomen bedeutet, das gegebenenfalls durch Phenyl substituiert ist

und deren Salze.

Physiologisch unbedenkliche Salze sind Salze der erfindungsgemäßen Verbindungen mit anorganischen oder organischen Säuren. Bevorzugt werden Salze mit anorganischen Säuren wie beispielsweise Salzsäure, Bromwasserstoffsäure, Phosphorsäure oder Schwefelsäure, oder Salze mit organischen Carbon- oder Sulfonsäuren wie beispielsweise Essigsäure, Maleinsäure, Fumarsäure, Äpfelsäure, Zitronensäure, Weinsäure, Milchsäure, Benzoesäure, oder Methansulfonsäure, Ethansulfonsäure, Phenylsulfonsäure, Toluolsulfonsäure oder Naphthalindisulfonsäure.

Die erfindungsgemäßen Verbindungen existieren in stereoisomeren Formen, die sich entweder wie Bild und Spiegelbild (Enantiomere), oder die sich nicht wie Bild und Spiegelbild (Diastereomere) verhalten. Die Erfindung betrifft sowohl die Antipoden als auch die Racemformen sowie die Diastereomerengemische. Die Racemformen lassen sich ebenso wie die Diastereomeren in bekannter Weise in die stereoisomer einheitlichen Bestandteile trennen.

Bevorzugt sind Verbindungen der allgemeinen Formel (I)

in welcher,

$R^1$ für geradkettiges oder verzweigtes Alkyl mit bis zu 5 Kohlenstoffatomen steht, das gegebenenfalls durch Cyclopropyl, Cyclopentyl oder Cyclohexyl substituiert ist, oder für Cyclopropyl, Cyclopentyl oder Cyclohexyl steht,

$R^2$ und $R^3$ gleich oder verschieden sind und für Fluor, Chlor, Brom, Cyano oder Trifluormethyl stehen,

oder

$R^2$ oder $R^3$ für Wasserstoff steht,

und

$R^4$ für geradkettiges oder verzweigtes Alkyl mit bis zu 5 Kohlenstoffatomen steht, das durch Acetyl, Methoxycarbonyl oder durch einen Rest der Formel

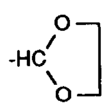 , 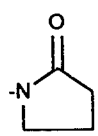 , 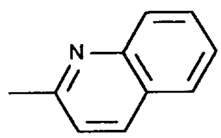 , 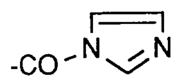 ,

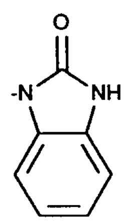 , 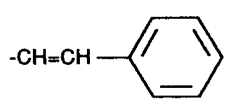 , 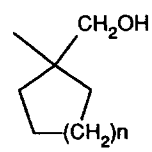

oder -CO-NH-$R^5$

substituiert ist,

worin

n eine Zahl 1 oder 2 bedeutet, und

$R^5$ Cyclopentyl, Cyclohexyl oder Cycloheptyl, bedeutet, oder geradkettiges oder verzweigtes Alkyl mit bis zu 5 Kohlenstofatomen bedeutet, das gegebenenfalls durch Phenyl substituiert ist

und deren Salze.

Besonders bevorzugt sind Verbindungen der allgemeinen Formel (I),

in welcher

$R^1$ für geradkettiges oder verzweigtes Alkyl mit bis zu 5 Kohlenstoffatomen steht, das gegebenenfalls durch Cyclopentyl oder Cyclohexyl substituiert ist, oder für Cyclopropyl, Cyclopentyl oder Cyclohexyl steht,

$R^2$ und $R^3$ gleich oder verschieden sind und für Fluor, Chlor, Brom, Cyano oder Trifluormethyl stehen,

oder

$R^2$ oder $R^3$ für Wasserstoff steht,

und

$R^4$ für geradkettiges oder verzweigtes Alkyl mit bis zu 4 Kohlenstoffatomen steht, das durch Acetyl, Methoxycarbonyl oder durch einen Rest der Formel

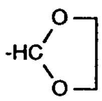 , 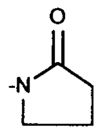 , 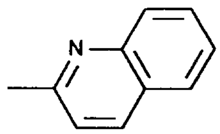 , 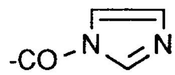 ,

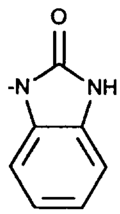 , 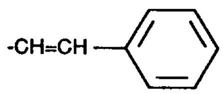 , 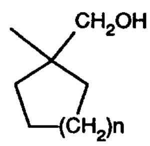

oder $-CO-NH-R^5$
substituiert ist,
worin
n eine Zahl 1 oder 2 bedeutet
und
$R^5$ Cyclopentyl oder Cyclohexyl bedeutet, oder geradkettiges oder verzweigtes Alkyl mit bis zu 4 Kohlenstoffatomen bedeutet, das gegebenenfalls durch Phenyl substituiert ist
und deren Salze.

Außerdem wurden Verfahren zur Herstellung der erfindungsgemäßen Verbindungen der allgemeinen Formel (I) gefunden, dadurch gekennzeichnet, daß man
[A] Verbindungen der allgemeinen Formel (II)

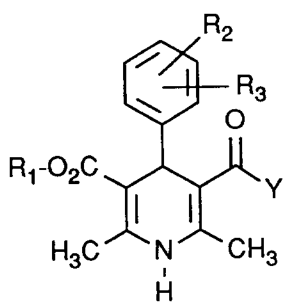 (II)

in welcher
$R^1$, $R^2$ und $R^3$ die angegebene Bedeutung haben
und
Y zusammen mit der -CO-Gruppe eine reaktives Carbonsäurederivat bildet,
in inerten Lösemitteln, in Anwesenheit einer Base,
mit Verbindungen der allgemeinen Formel (III)

$R^4$-OH (III)

in welcher
$R^4$ die angegebene Bedeutung hat,
umsetzt,
oder
[B] im Fall, daß $R^4$ für die Gruppe der Formel

-A-CO-NH-$R^5$ steht,

worin
$R^5$ die oben angegebene Bedeutung hat und A, wie oben unter $R^4$ definiert, für $C_1$-$C_6$-Alkyl steht, entweder Verbindungen der allgemeinen Formel (IV)

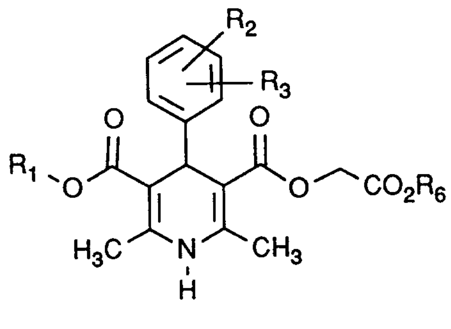
(IV)

in welcher
$R^1$, $R^2$ und $R^3$ die angegebene Bedeutung haben
und
$R^6$ für $C_1$-$C_4$-Alkyl steht,
zunächst zu den entsprechenden Carbonsäuren ($R^6$ = H) verseift, anschließend diese gegebenenfalls in ein reaktives Carbonsäurederivat der allgemeinen Formel (IIa)

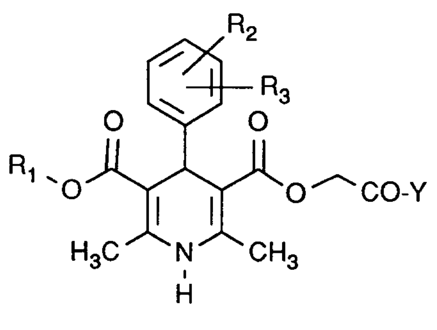
(IIa)

in welcher
$R^1$, $R^2$, $R^3$ und Y die angegebene Bedeutung haben,
überführt, und in einem letzen Schritt mit Aminen der allgemeinen Formel (V)

$H_2N$-$R^5$ (V)

in welcher
$R^5$ die angegebene Bedeutung hat,
umsetzt,
oder daß man Carbonsäuren der allgemeinen Formel (VI)

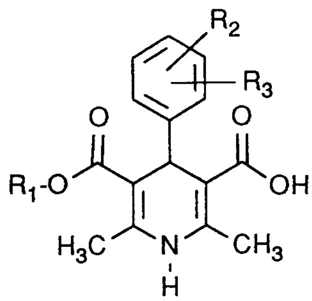

(VI)

in welcher
$R^1$, $R^2$ und $R^3$ die angegebene Bedeutung haben,
mit Verbindungen der allgemeinen Formel (VII)

$R^4$-B (VII)

in welcher
$R^4$ die oben angegebene Bedeutung hat und
B für Halogen, vorzugsweise für Brom steht,
in Gegenwart einer geeigneten Base in einem inerten Lösemittel, alkylierend verestert,
und im Fall, der reinen Enantiomere, die enantiomerenreinen Carbonsäuren, gegebenenfalls zunächst über die Stufe eines reaktiven Säurederivates, mit den entsprechenden Alkoholen umsetzt.

Das erfindungsgemäße Verfahren [A] kann durch folgendes Formelschema beispielhaft erläutert werden:

[A]

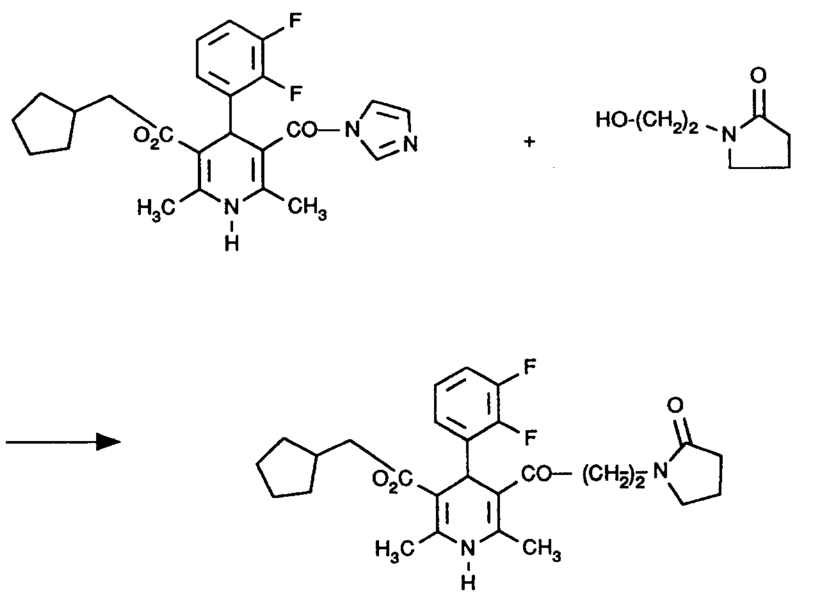

Als Lösemittel für die Verfahren eignen sich hierbei alle inerten organischen Lösemittel, die sich unter den Reaktionsbedingungen nicht verändern. Hierzu gehören bevorzugt Ether wie Diethylether, Dioxan, Tetrahydrofuran, Glykoldimethylether, oder Diethylenglykoldimethylether, Acetonitril, oder Amide wie Hexamethylphosphorsäuretriamid oder Dimethylformamid, oder halogenierte Kohlenwasserstoffe wie Methylenchlorid, Tetrachlorkohlenstoff oder Kohlenwasserstoffe wie Benzol oder Toluol. Ebenso ist es möglich, Gemische der genannten Lösemittel zu verwenden. Bevorzugt sind Toluol, Tetrahydrofuran, Dioxan und Dimethylformamid.

Als Basen für die Aktivierung der Carbonsäuren eignen sich im allgemeinen Alkalihydride- oder -alkoholate, wie beispielsweise Natriumhydrid oder Kalium-tert.butylat, oder cyclische Amine, wie beispielsweise Piperidin, Dimethylaminopyridin oder $C_1$-$C_4$-Alkylamine, wie beispielsweise Triethylamin. Bevorzugt sind in Abhängigkeit der jeweiligen Reaktionsschritte Piperidin, Dimethylaminopyridin, Pyridin, Natriumhydrid und Kalium-tert.butylat.

Als Hilfsstoffe werden bevorzugt Kondensationsmittel eingesetzt die auch Basen sein können. Bevorzugt werden hier die üblichen Kondensationsmittel wie Carbodiimide z.B. N,N'-Diethyl-, N,N'-Dipropyl-, N,N'-Diisopropyl-, N,N'-Dicyclohexylcarbodiimid, oder N-(3-Dimethylaminoisopropyl)-N'-ethylcarbodiimid-Hydrochlorid, oder Carbonylverbindungen wie Carbonyldiimidazol, oder 1,2-Oxazoliumverbindungen wie 2-Ethyl-5-phenyl-1,2-oxazolium-3-sulfonat oder 2-tert.-Butyl-5-methyl-isoxazolium-perchlorat, oder Acylaminoverbindungen wie 2-Ethoxy-1-ethoxycarbonyl-1,2-dihydrochinolin, oder Propanphosphonsäureanhydrid, oder Isobutylchloroformat, oder Benzotriazolyloxy-tris(dimethylamino)phosphonium-hexafluorophosphonat. Bevorzugt sind N,N'-Dicyclohexylcarbodiimid und Carbonyldiimidazol.

Als Basen eignen sich im allgemeinen Alkalicarbonate wie beispielsweise Natrium-oder Kaliumcarbonat, oder organische Basen wie Trialkylamine, z.B. Triethylamin, N-Ethylmorpholin, N-Methylpiperidin oder Diisopropylethylamin, oder Dimethylaminopyridin, 1,8-Diazabicyclo[5.4.0]undec-7-en (DBU) oder 1,5-Diazabicyclo[4.3.0]non-5-en (DBN). Bevorzugt ist Dimethylaminopyridin.

Die Base wird im allgemeinen in einer Menge von 0,01 mol bis 1 mol, bevorzugt von 0,05 mol bis 0,1 mol jeweils bezogen auf 1 mol der Verbindungen der allgemeinen Formeln (II) oder (VI) eingesetzt.

Die Hilfsstoffe werden im allgemeinen in einer Menge von 1 mol bis 3 mol, bevorzugt von 1 mol bis 1,5 mol, jeweils bezogen auf 1 mol der Verbindungen der allgemeinen Formeln (II), (IV) und (VI) eingesetzt.

Die Reaktionstemperaturen können in einem größeren Bereich variiert werden. Im allgemeinen arbeitet man in einem Bereich von -20 °C bis 200 °C, bevorzugt von 0 °C bis 25 °C.

Die Verfahren können bei Normaldruck, erhöhtem oder erniedrigtem Druck (beispielsweise von 0,5 bis 5 bar), vorzugsweise bei Normaldruck durchgeführt werden.

Bei der Durchführung der erfindungsgemäßen Verfahren ist das Verhältnis der an der Reaktion beteiligten Stoffe beliebig. Im allgemeinen arbeitet man jedoch bei molaren Mengen der Reaktanden.

Enantiomerenreine Formen erhält man z.B. außerdem, indem man Diastereomerengemische der Verbindungen der allgemeinen Formel (I), in welcher $R^1$ oder $R^4$ für einen chiralen Esterrest steht, nach üblicher Methode trennt, anschließend die enantiomerenreinen Carbonsäuren herstellt und dann gegebenenfalls durch Veresterung mit entsprechenden Alkoholen in die enantiomerenreinen Dihydropyridine überführt.

Geeignet als chirale Esterreste sind alle Ester enantiomerenreiner Alkohole wie beispielsweise 2-Butanol, 1-Phenylethanol, Milchsäure, Milchsäureester, Mandelsäure, Mandelsäureester, 2-Aminoalkohole, Zuckerderivate und viele andere enantiomerenreine Alkohole mehr.

Die Trennung der Diastereomeren erfolgt im allgemeinen entweder durch fraktionierte Kristallisation, durch Säulenchromatographie oder durch Craig-Verteilung. Welches das optimale Verfahren ist, muß von Fall zu Fall entschieden werden, manchmal ist es auch zweckmäßig, Kombinationen der einzelnen Verfahren zu benutzen. Besonders geeignet ist die Trennung durch Kristallisation oder Craig-Verteilung bzw. eine Kombination beider Verfahren.

Die Veresterung der enantiomerenreinen Dihydropyridine erfolgt bevorzugt in Ethern wie Diethylether oder Tetrahydrofuran, Dimethylformamid, Methylenchlorid, Chloroform, Acetonitril oder Toluol.

Die reaktiven Säurederivate der allgemeinen Formel (II) und (IIa) sind teilweise bekannt oder neu und können dann nach üblichen Methoden über die entsprechenden Carbonsäuren hergestellt werden.

Ebenso sind die Alkohole der allgemeinen Formel (III) und die Amine der allgemeinen Formel (V) bekannt.

Die Verbindungen der allgemeinen Formeln (IV) und (VI) sind teilweise bekannt oder neu und können dann aber nach publizierten Verfahren hergestellt werden.

Die vorstehenden Herstellungsverfahren sind lediglich zur Verdeutlichung angegeben. Die Herstellung der Verbindungen der allgemeinen Formel (I) ist nicht auf diese Verfahren beschränkt, sondern jede Modifikation dieser Verfahren ist in gleicher Weise für die Herstellung der erfindungsgemäßen Verbindungen anwendbar.

Die erfindungsgemäßen Verbindungen zeigen ein nicht vorhersehbares, wertvolles pharmakologisches Wirkspektrum.

Die erfindungsgemäßen Verbindungen sind Calciumkanalliganden mit Selektivität für L-Typ-Calciumkanäle des zentralen Nervensystems. Diese Selektivität läßt sich z.B. durch Vergleich der Bindungsaffinitäten zu DHP-Bindungstellen an Rattenhirn und Rattenherz zeigen.

Die Verbindungen beeinflussen positiv Lern- und Gedächtnisleistungen, wie ihre leistungsverbessernde Wirkung in typischen Lern- und Gedächtnismodellen wie Wasser-Labyrinth, Morris-Labyrinth, Passives Vermeiden oder Reminiszenztests in automatisierten Skinner-Boxen demonstriert.Sie besitzen ein antidepressives Potential, wie ihre Wirksamkeit im Rattenschwimmtest nach Porsolt belegt.

Bindungsassays:

Die Bindungsaffinitäten zu PN 200-110-Bindungsstellen im Rattenhirnen bzw. Rattenherzen wurden nach Rampe D.R., Mutledge A., Janis R.A., Triggle D.J.: Can. Journ. Physiol. Pharmacol. 65, (1987) 1452 bestimmt.

Wasserlabyrinth:

Alte Wistar-Ratten werden an die Startposition in einem mit kaltem Wasser gefüllten, durch senkrechte Barrieren unterteilten Plastiktank gesetzt. Um zu einer Leiter zu gelangen, die den Tieren das Entkommen aus dem Wasser ermöglicht, müssen sie um diese Barrieren herumschwimmen. Die Zeit, die zum Auffinden des Ausstiegs benötigt wird und die Zahl der Fehler auf dem Weg dorthin werden aufgezeichet. Dabei wird ein Fehler definiert als Schwimmen in eine Sackgasse oder Schwimmen über die Grenzlinie imaginärer Quadrate, in die der Tank unterteilt ist, in die Richtung fort vom Ausstieg.
Die Ratten bleiben bis zum Finden des Ausgangs, längstens aber 300 sec. im Labyrinth. Dann werden sie aufgenommen, getrocknet und unter einem Rotlicht gewärmt. Danach kehren sie in ihre Heimatkäfige zurück.
In einem typischen Experiment werden durch einen Vortest zwei äquivalente Tiergruppen (Placebo, Testsubstanz je n = 15) ermittelt. Anschließend durchlaufen die Tiere 6 Testsitzungen, zwei je Tag. Testsubstanzen bzw. Placebo werden 30 min. vor den Versuchen per os verabreicht. Maß für die lern- und gedächtnisverbessernde Wirkung der Testsubstanzen im Vergleich zu Placebo sind Verkürzung der Zeit bis zum Erreichen des Ausstiegs, Verringerung der Zahl der Fehler und Vergrößerung der Zahl der Tiere,die den Ausstieg überhaupt finden.

Rattenschwimmtest nach Porsolt

Während eines Vortests werden junge Ratten in einem Glaszylinder (40 cm hoch, 20 cm Durchmesser) gesetzt, der 17 cm hoch mit Wasser von 25 °C gefüllt ist. Nach 20 min im Wasser werden die Tiere herausgenommen und unter einer Lampe 30 min gewärmt. In diesem Vortest versuchen alle Ratten, aus dem Zylinder zu entkommen, bis sie nach etwa 15 min immobil verharren ("behavioral despair", Aufgabeverhalten). 24 h später beginnt die Testsitzung in der die Ratten wie am Vortag in den Glaszylinder gesetzt werden, jedoch diesmal nur 5 min. Die Zeitspannen, die die Ratten während dieser 5 min immobil verharren, werden aufgezeichnet. Dabei wird eine Ratte als immobil angesehen, die aufrecht im Wasser treibend nur noch minimale Bewegungen ausführt, um den Kopf über Wasser zu halten. Die antidepressive Wirkung der Testsubstanzen zeigt sich in der Reduktion der Immobilitätsdauer im Vergleich zu den Placebowerten.

Aufgrund ihrer pharmakologischen Eigenschaften können die erfindungsgemäßen Verbindungen für die Herstellung von Arzneimitteln zur Behandlung von zentral degenerativen Erkrankungen eingesetzt werden, wie z.B. Auftreten bei Demenzen (Multiinfarktdemenz, MID, primär degenerativer Demenz PDD, prä- und seniler Alzheimerscher Krankheit, HIV-Demenz und andere Demenzformen), Parkinsonscher Krankheit oder amyotrophischer Lateralsklerose.

Weiterhin eignen sich die Wirkstoffe zur Behandlung von Hirnleistungsstörungen im Alter, des hirnorganischen Psychosyndroms (HOPS, Organic brain syndrom, OBS) und von altersbedingten Gedächtnisstörungen (age associated memory impairment, AAMI).

Sie sind wertvoll zur Prophylaxe und zur Bekämpfung der Folgen cerebraler Durchblutungsstörungen wie cerebraler Ischämien, Schlaganfällen und von Subarachnoidalblutungen.

Sie sind geeignet zur Behandlung von Depressionen und der Manie. Weitere Anwendungsgebiete sind die Behandlung von Migräne, von Neuropathien, von Suchterkrankungen und Entzugserscheinungen.

Zur vorliegenden Erfindung gehören auch pharmazeutische Zubereitungen, die neben inerten, nicht-toxischen, pharmazeutisch geeigneten Hilfs- und Trägerstoffen eine oder mehrere Verbindungen der allgemeinen Formel (I) enthalten, oder die aus einem oder mehreren Wirkstoffen der Formel (I) bestehen, sowie Verfahren zur Herstellung dieser Zubereitungen.

Die Wirkstoffe der Formel (I) sollen in diesen Zubereitungen in einer Konzentration von 0,1 bis 99,5 Gew.-%, bevorzugt von 0,5 bis 95 Gew.-% der Gesamtmischung vorhanden sein.

Neben den Wirkstoffen der Formel (I) können die pharmazeutischen Zubereitungen auch andere pharmazeutische Wirkstoffe enthalten.

Die oben aufgeführten pharmazeutischen Zubereitungen können in üblicher Weise nach bekannten Methoden hergestellt werden, beispielsweise mit dem oder den Hilfs- oder Trägerstoffen.

Im allgemeinen hat es sich als vorteilhaft erwiesen, den oder die Wirkstoffe der Formel (I) in Gesamtmengen von etwa 0,01 bis etwa 70 mg/kg, bevorzugt in Gesamtmengen von etwa 0,1 mg/kg bis 20 mg/kg Körpergewicht je 24 Stunden, gegebenenfalls in Form mehrerer Einzelgaben, zur Erzielung des gewünschten Ergebnisses zu verabreichen.

Es kann aber gegebenenfalls vorteilhaft sein, von den genannten Mengen abzuweichen, und zwar in Abhängigkeit von der Art und vom Körpergewicht des behandelten Objekts, vom individuellen Verhalten gegenüber dem Medikament, der Art und Schwere der Erkrankung, der Art der Zubereitung und Applikation, sowie dem Zeitpunkt bzw. Intervall, zu welchem die Verabreichung erfolgt.

Die jeweils aufgeführten $R_f$-Werte wurden - sofern nicht anders vermerkt - durch Dünnschichtchromatographie auf Kieselgel (Alufolie, Kieselgel 60 F 254, Fa. E. Merck) ermittelt. Die Visualisierung der Substanzflecke geschah durch Betrachten unter UV-Licht und/oder durch Besprühen mit 1%iger Kaliumpermanganat-Lösung und/oder Molybdatophosphorsäure.

Die Flash-Chromatographie wurde auf Kieselgel 60, 0,040 - 0,064 mm, Fa. E. Merck, durchgeführt. Elution mit Lösemittelgradienten bedeutet: Beginnend mit der reinen, unpolaren Lösemittelgemischkomponente wird in einem steigendem Ausmaß die polare Laufmittelkomponente zugemischt, bis das gewünschte Produkt eluiert wird (DC-Kontrolle).

Ausgangsverbindungen

Beispiel I

(Carboxymethyl)-(isopropyl)-4-(2-chlorphenyl)-1,4-dihydro-2,6-dimethylpyridin-3,5-dicarboxylat

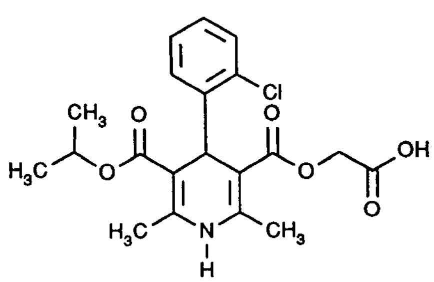

Zunächst werden 13,9 g (34,8 mmol) 4-(2-Chlorphenyl)-1,4-dihydro-5-(1-imidazolylcarbonyl)-2,6-dimethylpyridin-3-carbonsäureisopropylester, 3,1 g (34,8 mmol) Glycolsäuremethylester und 1,04 g (34,8 mmol) Natriumhydrid-Dispersion in Paraffinöl (80%) in insgesamt 225 ml trockenem Tetrahydrofuran zu (Isopropyl)(methoxycarbonylmethyl)-4-(2-chlorphenyl)-1,4-dihydro-2,4-dimethylpyridin-3,5-dicarboxylat umgesetzt. Dieses wird ohne Isolierung durch Zutropfen von 30 ml Wasser und 7 ml 1 n Natronlauge in die Reaktionslösung in einer 18stündigen Reaktionszeit bei Raumtemperatur vollständig verseift. Danach wird die Reaktionslösung in ein Gemisch aus 1 l 5%iger Natriumchloridlösung und 500 ml Toluol eingerührt, und durch Zutropfen von 35 ml 1 n Salzsäure auf pH 6 - 7 eingestellt. Die wäßrige Phase wird noch einmal mit 250 ml Toluol extrahiert. Danach verrührt man die wäßrige Phase wieder mit einem Gemisch aus 400 ml Toluol und 100 ml Tetrahydofuran und stellt mit weiteren 40 ml 1 n Salzsäure auf pH 3. Die organische Phase wird abgetrennt und die wäßrige Phase noch einmal mit 250 ml Toluol nachextrahiert. Die beiden letzten (sauren) organischen Phasen werden vereinigt, zweimal mit 150 ml Wasser gewaschen und über Natriumsulfat getrocknet. Nachdem man das Lösemittel unter Vakuum abdestilliert hat, verbleiben 11,0 g (78% d.Th.) Zielverbindung.
DC: auf Kieselgel/Alufolie (Merck Art. 5549) mit Toluol/Methanol 70:30
$R_F$ = 0,23

Herstellungsbeispiele

Beispiel 1

(Cyclopentylmethyl)-[2-(2-oxopyrrolidin)ethyl]-4-(2,3-difluorphenyl)-1,4-dihydro-2,6-dimethylpyridin-3,5-dicarboxylat

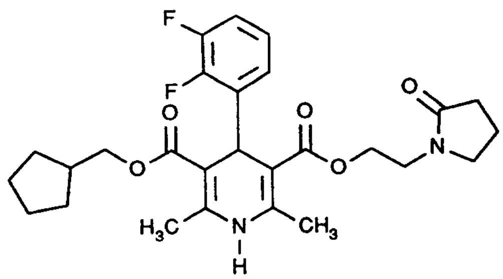

210 mg (7 mmol) Natriumhydrid-Dispersion in Paraffinöl (80%ig) werden in Petroleumbenzin ausgewaschen und in 15 ml trockenem Tetrahydrofuran suspendiert. Dazu wird innerhalb von 30 min bei 0°C bis +5°C eine Lösung von 3,1 g (7 mmol) 4-(2,3-Difluorphenyl)-1,4-dihydro-5-(1-imidazolylcarbonyl)-2,6-dimethylpyridin-3-carbonsäurecyclopentylmethylester in 15 ml trockenem Tetrahydofuran getropft und 30 min nachreagieren gelassen, bis die Wasserstoffentwicklung beendet ist. Anschließend wird die Reaktionslösung auf 40°C erwärmt und innerhalb von 30 min eine Lösung von 0.90 g (7 mmol) 1-(2-Hydroxyethyl)-2-pyrrolidinon in 15 ml trockenem Tetrahydrofuran zugetropft. Nach einer einstündigen Reaktionszeit wird die Reaktionslösung in ein Gemisch aus 220 ml 5%iger Natriumchloridlösung und 110 ml Toluol eingerührt und die wäßrige Phase noch einmal mit 55 ml Toluol extrahiert. Die vereinigten organischen Phasen werden zweimal mit 55 ml Wasser gewaschen und über Natriumsulfat getrocknet. Nachdem man das Lösemittel unter Vakuum abdestilliert hat, verbleiben 3,0 g öliges Rohprodukt. Das Rohprodukt wird auf einer Kieselgel-Säule mit Essigester / Ethanol 90:10 chromatographiert, und nach Einengen der Hauptfraktion auf ca. 10 ml kristallisiert das Endprodukt aus. Es wird noch 2 h auf 10 - 15°C gekühlt und danach wird das Kristallisat abgesaugt und getrocknet. Man erhält 2,1 g (59% der Theorie) der Titelverbindung mit einem Schmelzpunkt von 170-171°C.

Beispiel 2

[2-(1-Imidazolyl)-2-oxoethyl]-(isopropyl)-4-(2-chlorphenyl)-1,4-dihydro-2,6-dimethylpyridin-3,5-dicarboxylat

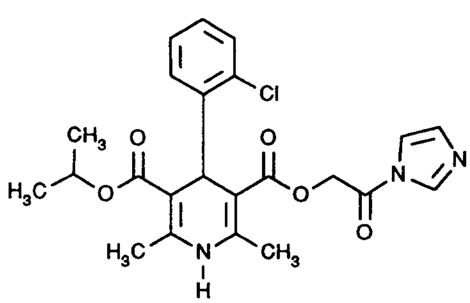

10,7 g (26,2 mmol) (Carboxymethyl)-(isopropyl)-4-(2-chlorphenyl)-1,4-dihydro-2,6-dimethylpyridin-3,5-dicarboxylat werden in 87 ml trockenem Tetrahydrofuran gelöst und die Lösung auf 50°C erwärmt. Dann wird innerhalb von 1 h eine Lösung aus 4,7 g (28,8 mmol) 1,1'-Carbonyldiimidazol und 70 ml trockenem Tetrahydrofuran zugetropft und 1 h weitergerührt, bis die Kohlendioxydentwicklung beendet ist. Nach Abkühlung wird die Reaktionslösung in eine Mischung aus 800 ml Wasser und 400 ml Toluol eingerührt. Die wäßrige Phase wird noch einmal mit 200 ml Toluol extrahiert. Nachdem die vereinigten organischen

Phasen zweimal mit 200 ml Wasser gewaschen und über Natriumsulfat getrocknet wurden, wird die Lösung unter Vakuum auf ca. 300 ml eingeengt. Das Endprodukt kristallisiert nun aus und wird nach 2stündiger Kühlung auf +10 bis 15 °C abgesaugt und getrocknet. Man erhält 9,0 g Kristallisat mit einem Schmelzpunkt von 167- 170 °C (Zers.).

Beispiel 3

(Isopropyl)-[2-oxo-2-(2-phenylethylamino)ethyl]-4-(2-chlorphenyl)-1,4-dihydro-2,6-dimethylpyridin-3,5-dicarboxylat

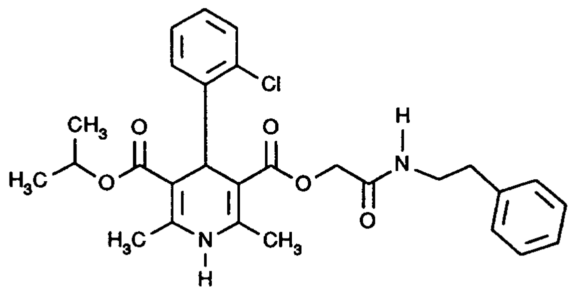

2,2 g (4,8 mmol) [2-(1-Imidazolyl)-2-oxoethyl]-(isopropyl)-4-(2-chlorphenyl)-1,4-dihydro-2,6-dimethylpyridin-3,5-dicarboxylat werden in 22 ml Tetrahydrofuran gelöst. Innerhalb von 30 min wird eine Lösung von 0,64 g (5,3 mmol) 2-Phenylethylamin bei 20-25 °C zugetropft. Anschließend wird die Reaktionslösung noch 1 h bei Raumtemperatur gerührt, und dann in eine Mischung aus 160 ml 5%iger Natriumchloridlösung, 80 ml Toluol und 40 ml Tetrahydrofuran (60 ml) eingerührt. Die wäßrige Phase wird noch einmal mit Toluol/Tetrahydrofuran 1:1(60 ml) extrahiert. Nachdem die vereinigten organischen Phasen zweimal mit je 60 ml Wasser gewaschen, und über Natriumsulfat getrocknet wurden, wird die Lösung auf ca. 50 ml eingeengt. Das Endprodukt kristallisiert nun aus und wird nach 1/2stündiger Kühlung auf ca. 10-15 °C abgesaugt und getrocknet. Man erhält 2,2 g (90% d.Th.) Kristallisat mit einem Schmelzpunkt von 163-164 °C.

In Analogie zu den Vorschriften der Beispiele I, 1, 2 und 3 werden die in Tabelle 1 aufgeführten Verbindungen hergestellt:

Tabelle 1:

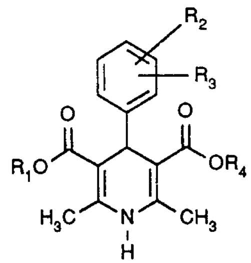

| Bsp.-Nr. | $R^1$ | $R^2$ | $R^3$ | $R^4$ | F°C | Herstellung analog Beispiel-Nr. |
|---|---|---|---|---|---|---|
| 4 | $-CH(CH_3)_2$ | 2-Cl | H | $-CH_2-CO-NH-CH_2-CH(CH_3)_2$ | 192-194 | 4 |
| 5 | $-CH(CH_3)_2$ | 2-Cl | H | $-CH_2-CO-NH-$ 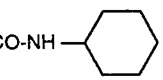 | 202-205 | 4 |
| 6 | $-CH(CH_3)_2$ | 2-Cl | H | $-CH_2-CO-NH-CH_2-$ 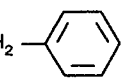 | 209-210 | 4 |
| 7 | $-CH(CH_3)_2$ | 2-Cl | H | $-CH_2-CO-NH-(CH_2)_2-$ 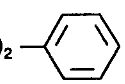 | 163-164 | 4 |
| 8 | 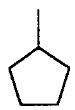 | 2-F | 3-F | $-(CH_2)_2-$ 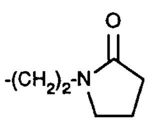 | 194-195 | 1 |
| 9 | 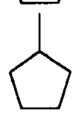 | 2-F | 3-F | $-CH_2-CO_2CH_3$ | 129-130 | 1 |
| 10 | $-CH(CH_3)_2$ | 2-F | 3-F | $-(CH_2)_2-$ 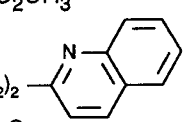 | 175 | 1 |
| 11 | $-CH(CH_3)_2$ | 2-F | 3-F | $-(CH_2)_2-$ 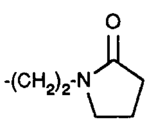 | 188 | 1 |

Fortsetzung Tabelle 1:

| Bsp.-Nr. | $R^1$ | $R^2$ | $R^3$ | $R^4$ | F°C | Herstellung analog Beispiel-Nr. |
|---|---|---|---|---|---|---|
| 12 | $-CH(CH_3)_2$ | 2-F | 3-F | 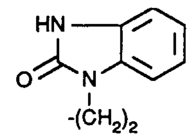 | 200 | 1 |
| 13 | $-CH(CH_3)_2$ | 2-F | 3-F | 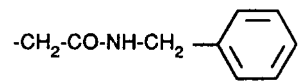 | 185 | 4 |
| 14 | $-CH(CH_3)_2$ | 2-F | 3-F | $-CH_2-CO_2CH_3$ | 112 | 1 |
| 15 | $-CH(CH_3)_2$ | 2-F | 3-F | 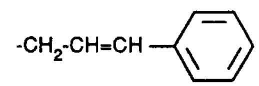 | 0,22a) | 1 |
| 16 | $-CH_3$ | 2-F | 3-F | 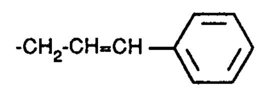 | 139 | 1 |
| 17 | $-CH_3$ | 2-F | 3-F | 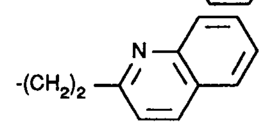 | 207 | 1 |
| 18 | $-CH_3$ | 2-F | 3-F | 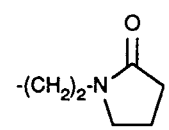 | 200 | 1 |
| 19 | 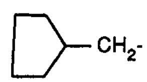 | 2-F | 3-F | 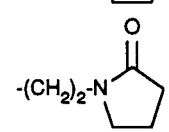 | 170-171 | 1 |

<u>Fortsetzung Tabelle 1</u>:

| Bsp.-Nr. | $R^1$ | $R^2$ | $R^3$ | $R^4$ | F°C | Herstellung analog Beispiel-Nr. |
|---|---|---|---|---|---|---|
| 20 | 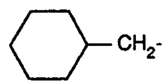 | 2-F | 3-F | 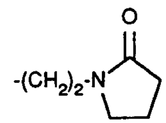 | 163-164 | 1 |
| 21 | $-CH(CH_3)_2$ | 2-Cl | H | 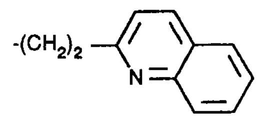 | 131 | 1 |
| 22 | $-CH(CH_3)_2$ | 2-Cl | H | 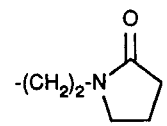 | 183 | 1 |
| 23 | $-CH(CH_3)_2$ | 2-Cl | H | 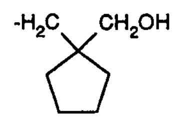 | 137-138 | 1 |
| 24 | $-CH(CH_3)_2$ | 2-Cl | H | 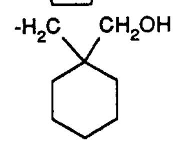 | 139-140 | 1 |
| 25 | $-CH(CH_3)_2$ | 2-F | 3-F | 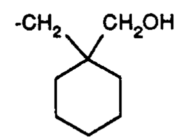 | 129 | 1 |

*) Toluol:Essigester 5/1

Beispiel 26

(Isopropyl)-(methoxycarbonylmethyl)-4-(2-chlor-6-fluorphenyl)-1,4-dihydro-2,6-dimethylpyridin-3,5-dicarboxylat

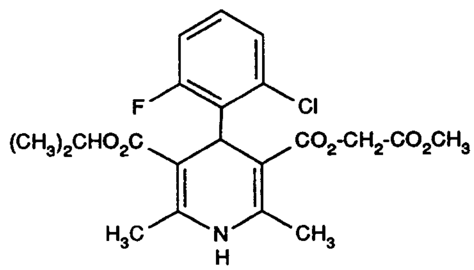

1,2 g (3,2 mmol) 4-(2-Chlor-6-fluorphenyl)-1,4-dihydro-2,6-dimethylpyridin -3,5-dicarbonsäuremonoisopropylester werden 10 min in 20 ml Dimethylformamid mit 0,28 g (2,0 mmol) Kaliumcarbonat gerührt. Man gibt 0,37 ml (3,9 mmol) Bromessigsäuremethylester zu und rührt 5 h bei Raumtemperatur. Man gießt auf 250 ml Wasser, extrahiert dreimal mit Essigester, wäscht die vereinigten organischen Phasen mit Wasser und mit gesättigter Kochsalzlösung, trocknet über Natriumsulfat und engt ein. Das verbleibende Rohprodukt wird durch Chromatographie an Kieselgel in Dichlormethan/Essigester 8:1 gereinigt. Man erhält so 1,2 g (85 %) der Zielverbindung als amorphen Feststoff ($R_f$($SiO_2$; Toluol/Essigester 5:1) = 0,27).

In Analogie zur Vorschrift des Beispiels 26 werden die in Tabelle 2 aufgeführten Beispiele hergestellt.

Tabelle 2:

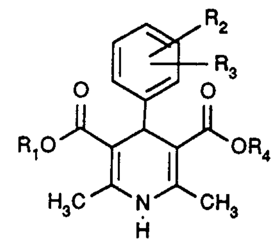

| Bsp.-Nr. | $R^1$ | $R^2$ | $R^3$ | $R^4$ | F°C | Herstellung analog Beispiel-Nr. |
|---|---|---|---|---|---|---|
| 27 | $-CH_3$ | 2-Cl | 3-Cl | 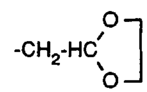 | 151 | |
| 28 | $-CH_3$ | 2-Cl | 3-Cl | 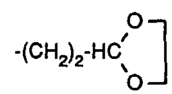 | 165-170 | |
| 29 | $-CH-(CH_3)_2$ | 2-Cl | 3-Cl | $-CH_2COCH_3$ | 121 | |
| 30 | 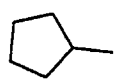 | 2-Cl | 3-Cl | $-CH_2COCH_3$ | 166 | |

*) Toluol:Essigester 5/1

## Patentansprüche

1. Dihydropyridine mit substituierten Estern der allgemeinen Formel

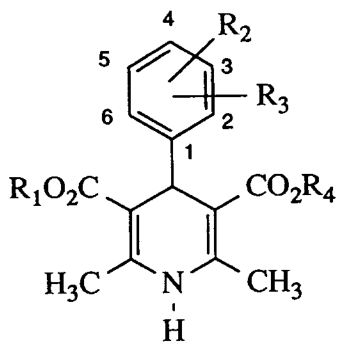

(I)

in welcher,

$R^1$ fuhr geradkettiges oder verzweigtes Alkyl mit bis zu 6 Kohlenstoffatomen steht, das gegebenenfalls durch Cycloalkyl mit 3 bis 6 Kohlenstoffatomen substituiert ist, oder für Cycloalkyl mit 3 bis 6 Kohlenstoffatomen steht,

$R^2$ und $R^3$ gleich oder verschieden sind und für Halogen, Cyano oder Trifluormethyl stehen,

oder

$R^2$ oder $R^3$ für Wasserstoff steht,

und

$R^4$ für geradkettiges oder verzweigtes Alkyl mit bis zu 6 Kohlenstoffatomen steht, das durch geradkettiges oder verzweigtes Acyl oder Alkoxycarbonyl mit jeweils bis zu 4 Kohlenstoffatomen oder durch einen Rest der Formel

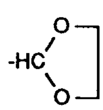 , 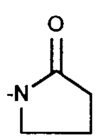 , 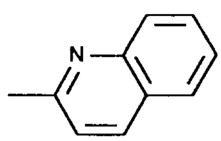 , 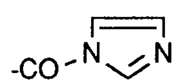 ,

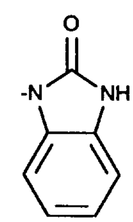 , 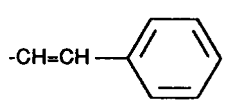 , 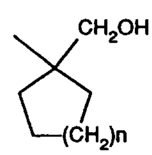

oder -CO-NH-$R^5$
substituiert ist,
worin

n eine Zahl 1, 2 oder 3 bedeutet

und

$R^5$ Cycloalkyl mit 3 bis 7 Kohlenstoffatomen bedeutet, oder geradkettiges oder verzweigtes Alkyl mit bis zu 6 Kohlenstoffatomen bedeutet, das gegebenenfalls durch Phenyl substituiert ist

und deren Salze.

2. Dihydropyridine mit substituierten Estern nach Anspruch 1 wobei

$R^1$ für geradkettiges oder verzweigtes Alkyl mit bis zu 5 Kohlenstoffatomen steht, das

gegebenenfalls durch Cyclopropyl, Cyclopentyl oder Cyclohexyl substituiert ist, oder für Cyclopropyl, Cyclopentyl oder Cyclohexyl steht,

$R^2$ und $R^3$ gleich oder verschieden sind und für Fluor, Chlor, Brom, Cyano oder Trifluormethyl stehen,

oder

$R^2$ oder $R^3$ für Wasserstoff steht,

und

$R^4$ für geradkettiges oder verzweigtes Alkyl mit bis zu 5 Kohlenstoffatomen steht, das durch Acetyl, Methoxycarbonyl oder durch einen Rest der Formel

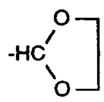 , 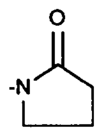 , 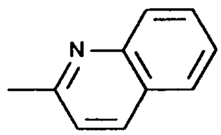 , 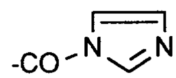 ,

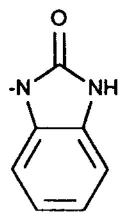 , 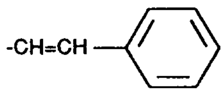 , 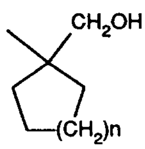

oder -CO-NH-$R^5$
substituiert ist,
worin

n eine Zahl 1 oder 2 bedeutet, und

$R^5$ Cyclopentyl, Cyclohexyl oder Cycloheptyl, bedeutet, oder geradkettiges oder verzweigtes Alkyl mit bis zu 5 Kohlenstoffatomen bedeutet, das gegebenenfalls durch Phenyl substituiert ist

und deren Salze.

**3.** Dihydropyridine mit substituierten Estern nach Anspruch 1,
wobei

$R^1$ für geradkettiges oder verzweigtes Alkyl mit bis zu 5 Kohlenstoffatomen steht, das gegebenenfalls durch Cyclopentyl oder Cyclohexyl substituiert ist, oder für Cyclopropyl, Cyclopentyl oder Cyclohexyl steht,

$R^2$ und $R^3$ gleich oder verschieden sind und für Fluor, Chlor, Brom, Cyano oder Trifluormethyl stehen,

oder

$R^2$ oder $R^3$ für Wasserstoff steht,

und

$R^4$ für geradkettiges oder verzweigtes Alkyl mit bis zu 4 Kohlenstoffatomen steht, das durch Acetyl, Methoxycarbonyl oder durch einen Rest der Formel

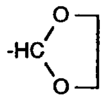 , 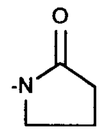 , 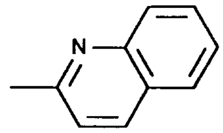 , 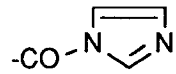 ,

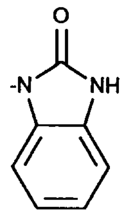 , 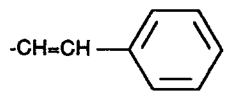 , 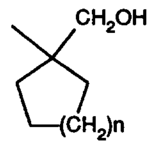

oder $-CO-NH-R^5$
substituiert ist,
worin

n eine Zahl 1 oder 2 bedeutet
und

$R^5$ Cyclopentyl oder Cyclohexyl bedeutet, oder geradkettiges oder verzweigtes Alkyl mit bis zu 4 Kohlenstoffatomen bedeutet, das gegebenenfalls durch Phenyl substituiert ist

und deren Salze.

**4.** Dihydropyridine mit substituierten Estern nach Anspruch 1 zur therapeutischen Anwendung.

**5.** Verfahren zur Herstellung von Dihydropyridinen mit substituierten Estern nach Anspruch 1 dadurch gekennzeichnet, daß man

[A] Verbindungen der allgemeinen Formel (II)

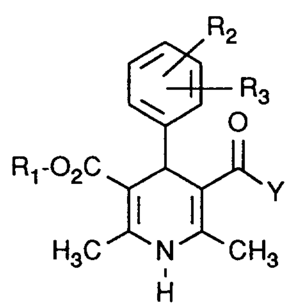 (II)

in welcher
$R^1$, $R^2$ und $R^3$ die angegebene Bedeutung haben
und

Y zusammen mit der -CO-Gruppe eine reaktives Carbonsäurederivat bildet,

in inerten Lösemitteln, in Anwesenheit einer Base,
mit Verbindungen der allgemeinen Formel (III)

$R^4$-OH (III)

in welcher

$R^4$ die angegebene Bedeutung hat,

umsetzt,
oder
[B] im Fall, daß $R^4$ für die Gruppe der Formel

-A-CO-NH-$R^5$ steht,

worin

$R^5$ die oben angegebene Bedeutung hat und A, wie oben unter $R^4$ definiert, für $C_1$-$C_6$-Alkyl steht,

entweder Verbindungen der allgemeinen Formel (IV)

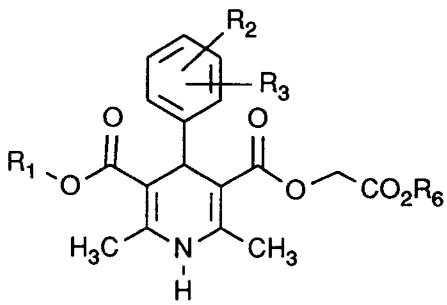

(IV)

in welcher

$R^1$, $R^2$ und $R^3$ die angegebene Bedeutung haben

und

$R^6$ für $C_1$-$C_4$-Alkyl steht,

zunächst zu den entsprechenden Carbonsäuren ($R^6$ = H) verseift, anschließend diese gegebenenfalls in ein reaktives Carbonsäurederivat der allgemeinen Formel (IIa)

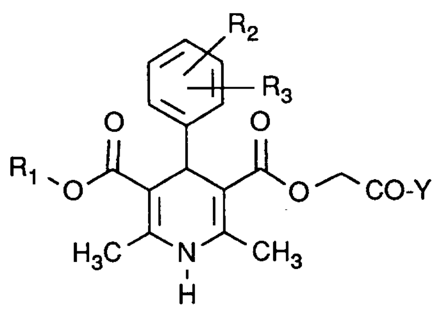

(IIa)

in welcher

$R^1$, $R^2$, $R^3$ und Y die angegebene Bedeutung haben,

überführt, und in einem letzen Schritt mit Aminen der allgemeinen Formel (V)

$H_2N$-$R^5$ (V)

in welcher

$R^5$ die angegebene Bedeutung hat,

umsetzt,

oder daß man Carbonsäuren der allgemeinen Formel (VI)

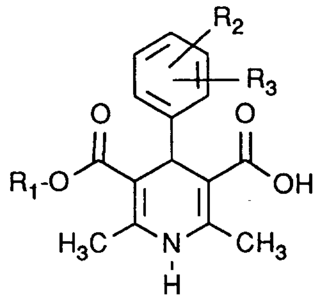

(VI)

in welcher
$R^1$, $R^2$ und $R^3$ die angegebene Bedeutung haben,
mit Verbindungen der allgemeinen Formel (VII)

$R^4$-B (VII)

in welcher
$R^4$ die oben angegebene Bedeutung hat und
B für Halogen, vorzugsweise für Brom steht,
in Gegenwart einer geeigneten Base in einem inerten Lösemittel, alkylierend verestert,
und im Fall, der reinen Enantiomere, die enantiomerenreinen Carbonsäuren, gegebenenfalls zunächst über die Stufe eines reaktiven Säurederivates, mit den entsprechenden Alkoholen umsetzt.

**6.** Arzneimittel enthaltend mindestens ein Dihydropyridin mit substituierten Estern nach Anspruch 1.

**7.** Arzneimittel nach Anspruch 6 zur Behandlung von zentral degenerativen Erkrankungen.

**8.** Arzneimittel nach Anspruch 6 zur Behandlung von Hirnleistungsstörungen, der Folgen cerebraler Ischämien sowie Depressionen.

**9.** Verwendung von Dihydropyridinen mit substituierten Estern nach Anspruch 1 zur Herstellung von Arzneimitteln.

**10.** Verfahren zur Herstellung von Arzneimitteln nach Anspruch 6 dadurch gekennzeichnet, daß man die Dihydropyridine gegebenenfalls mit Hilfe geeigneter Hilfs- und Trägerstoffe in eine geeignete Applikationsform überführt.

Europäisches Patentamt

# EUROPÄISCHER RECHERCHENBERICHT

Nummer der Anmeldung
EP 94 11 8678

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| A | EP-A-0 194 685 (NELSON RESEARCH & DEVELOPMENT)<br>* Ansprüche; Beispiele *<br>--- | 1-10 | C07D211/90<br>C07D401/12<br>C07D405/12<br>A61K31/44 |
| A | DE-A-22 18 644 (BAYER)<br>* Ansprüche; Beispiele *<br>--- | 1-10 | |
| A | EP-A-0 525 568 (BAYER)<br>* das ganze Dokument *<br>--- | 1-10 | |
| A | EP-A-0 494 816 (LABORATOIRE L. LAFON)<br>* das ganze Dokument *<br>----- | 1-10 | |
| | | | RECHERCHIERTE SACHGEBIETE (Int.Cl.6) |
| | | | C07D |

Der vorliegende Recherchenbericht wurde für alle Patentansprüche erstellt

| Recherchenort | Abschlußdatum der Recherche | Prüfer |
|---|---|---|
| DEN HAAG | 28. März 1995 | Zervas, B |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P04C03)